# FASCICULE DE BREVET EUROPEEN

(11) **EP 1 173 139 B1**
(45) Date de publication et mention de la délivrance du brevet: **08.10.2008**
(21) Numéro de dépôt: 01907688.4
(22) Date de dépôt: 24.01.2001
(51) Int. Cl.: A61K 8/88, A61Q 5/06

(54) **COMPOSITION SANS TRANSFERT STRUCTUREE SOUS FORME RIGIDE PAR UN POLYMERE**
DURCH EIN POLYMER IN EINER FESTEN FORM STRUKTURIERTE ABRIEBFESTE ZUSAMMENSETZUNG
TRANSFER-RESISTANT COMPOSITION STRUCTURED IN RIGID FORM BY A POLYMER

(30) Priorité: 24.01.2000 FR 0000920
(43) Date de publication de la demande: 23.01.2002
(73) Titulaire: L'ORÉAL, 75008 Paris (FR)
(72) Inventeur: FERRARI, Véronique, F-94700 Maisons-Alfort (FR)
(74) Mandataire: Le Coupanec, Pascale A.M.P.
(86) Numéro de dépôt international: PCT/FR2001/000229
(87) Numéro de publication internationale: WO 2001/052799

(56) Documents cités:
- EP-A- 0 820 764
- WO-A-95/24887
- WO-A-98/17243
- WO-A-98/17705
- US-A- 3 148 125

## Description

La présente invention se rapporte à une composition de soin et/ou de traitement et/ou de maquillage de la peau, y compris du cuir chevelu, et/ou des lèvres des êtres humains, contenant une phase grasse liquide renfermant un solvant volatil, structurée par un polymère particulier. Cette composition se présente notamment sous forme d'un stick de maquillage et plus spécialement de rouge à lèvres, dont l'application conduit à un dépôt brillant et sans transfert, remarquable.

Dans les produits cosmétiques ou dermatologiques, il est courant de trouver une phase grasse liquide structurée, à savoir gélifiée et/ou rigidifiée ; ceci est notamment le cas dans les compositions solides comme les déodorants, les baumes et les rouges à lèvres, les produits anti-cerne et les fonds de teint coulés. Cette structuration est obtenue à l'aide de cires et/ou de charges. Malheureusement, ces cires et charges ont tendance à matifier la composition, ce qui n'est pas toujours souhaitable en particulier pour un rouge à lèvres ; en effet, les femmes sont toujours à la recherche d'un rouge à lèvres sous forme d'un bâton déposant un film de plus en plus brillant.

Par "phase grasse liquide", au sens de l'invention, on entend une phase grasse liquide à température ambiante (25°C) et à pression atmosphérique (760 mm de Hg), composée d'un ou plusieurs corps gras liquides à température ambiante, appelés aussi huiles, généralement compatibles entre eux.

La structuration de la phase grasse liquide permet en particulier de limiter son exsudation des compositions solides notamment dans les régions chaudes et humides et, en plus, de limiter, après dépôt sur la peau ou les lèvres, la migration de cette phase dans les rides et ridules, ce qui est particulièrement recherché pour un rouge à lèvres ou un fard à paupières. En effet, une migration importante de la phase grasse liquide, en particulier lorsqu'elle est chargée de matières colorantes, conduit à un effet inesthétique autour des lèvres et des yeux, accentuant particulièrement les rides et les ridules. Cette migration est souvent citée par les femmes comme un défaut majeur des rouges à lèvres ou fard à paupières classiques.

La brillance est liée pour l'essentiel à la nature de la phase grasse liquide. Ainsi, il est possible de diminuer le taux de cires et de charges de la composition pour augmenter la brillance d'un rouge à lèvres mais alors, la migration de la phase grasse liquide augmente. Autrement dit, les taux de cires et de charges nécessaires à la réalisation d'un stick de dureté convenable et n'exsudant pas à température ambiante sont un frein à la brillance du dépôt.

Le demandeur a trouvé que la perte de brillance d'un stick contenant des cires était liée à la structure cristalline anisotrope de ces composés. Il a donc envisagé la fabrication d'un stick, en réduisant le taux de cire et/ou de charges.

De plus, la majorité des compositions de maquillage ou de soin, lorsqu'elles sont appliquées sur la peau, les cils ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition notamment de fond de teint ou de rouge à lèvres. Or à ce jour, les utilisateurs souhaitent embellir leur visage, y compris les lèvres, et leur corps en y passant le moins de temps possible. Par ailleurs, l'apparition de ces traces inacceptables notamment sur les cols de chemisier peut écarter certaines femmes de l'utilisation de ce type de maquillage.

Depuis plusieurs années, les cosméticiens se sont intéressés aux compositions de rouge à lèvres et plus récemment aux compositions de fond de teint "sans transfert". Ainsi, la société Shiseido a envisagé dans sa demande de brevet JP-A-61-65809 des compositions liquides de rouge à lèvres "sans transfert" contenant une résine siloxysilicate (à réseau tridimensionnel), une huile de silicone volatile à chaîne silicone cyclique et des charges pulvérulentes. De même la société Noevier à décrit dans le document JP-A-62-61911 des compositions de rouge à lèvres, d'eye-liner, de fonds de teint "sans transfert" comportant une ou plusieurs silicones volatiles associées à une ou plusieurs cires hydrocarbonées.

Ces compositions, bien que présentant des propriétés de "sans transfert" améliorées ont l'inconvénient de laisser sur les lèvres, après évaporation des huiles de silicone, un film qui devient inconfortable au cours du temps (sensation de dessèchement et de tiraillement), écartant un certain nombre de femmes de ce type de rouge à lèvres. En outre, le film déposé est mat.

Par ailleurs, la société Procter & Gamble a envisagé dans sa demande de brevet WO-A-96140044 des compositions de rouge à lèvres présentant des propriétés de "sans transfert" contenant une huile volatile et une huile non volatile du type perfluoropolyéther, incompatibles. Dans cette demande, il est, en outre, décrit l'amélioration de la brillance grâce à la dispersion préalable d'une phase huileuse dans une matrice, et la capacité de cette phase huileuse à ségréguer lors de l'application du produit sur le support et à migrer à la surface du film ainsi déposé.

Ce système a pour inconvénient de nécessité une bonne dispersion de la phase huileuse dans la matrice et peut engendrer des problèmes de stabilité du produit liés à la nécessaire mauvaise compatibilité de la phase huileuse avec la matrice (ségrégation de la composition dans son conditionnement). De plus, les huiles fluorées présentent l'inconvénient d'être délicates à formuler en particulier dans les milieux anhydres, limitant ainsi la gamme de produits cosmétiques réalisables industriellement.

La société Revlon a aussi envisagé dans le document US-A-5837223 d'associer un ester de Guerbet fluoré à une résine siloxysilicate et des solvants volatiles comme les silicones cycliques. La présence de résine de siloxysilicate conduit encore à des films mats inconfortables. De plus la présence d'huile fluorée rend la formulation des produits cosmétiques délicate. Dans le brevet US-A-5849275, la société Revlon a encore envisagé d'associer un polymère fluoré à des solvants volatiles comme les silicones cycliques. Là encore, la présence de composés fluorés rend la formulation des produits cosmétiques délicate.

Dans la demande EP-A-775483 de la société L'oréal, il est décrit des compositions de rouges à lèvres liquides contenant un milieu continue aqueux renfermant une dispersion de polymère capable de former un film continu brillant et « sans transfert » sur les lèvres. Malheureusement, ces compositions conduisent à un film sur les lèvres, sans cesse en mouvement, inconfortable et conférant une sensation de tiraillement. En outre, il est très difficile d'introduire des pigments dans ces compositions sans les déstabiliser.

Dans la demande EP-A-0749746 de la société L'oréal, il est décrit des compositions de rouges à lèvres contenant une dispersion de particules de polymère stabilisées en surface par un stabilisant polymérique. Ces compositions ont l'inconvénient de ne pouvoir contenir qu'une faible proportion d'huiles polaires connues pour apporter de la brillance au film déposé, dans des compositions classiques. En particulier, la présence d'une proportion importante d'huiles polaires (au moins 5 %) entraînent une floculation des particules et donc une instabilité dans le temps des compositions.

WO 98/17243, US 3,148,125 et WO 95/24887 décrivent des compositions gélifiées au moyen de polymères de type polyamide et présentant une stabilité améliorée, notamment au regard de la synérèse.

Il subsiste donc le besoin d'une composition ne présentant pas les inconvénients ci-dessus, et ayant notamment des propriétés de "sans transfert" remarquables, même lors d'une pression ou d'un frottement prononcé, de bonne tenue dans le temps, un aspect brillant, et ne desséchant pas la peau ou les lèvres sur lesquelles elle est appliquée, aussi bien lors de l'application qu'au cours du temps. De plus, cette composition est stable dans le temps, facile à fabriquer et l'introduction de pigments se fait aisément.

L'invention a justement pour objet une composition de soin et/ou de maquillage et/ou de traitement de la peau et/ou des lèvres du visage et/ou des phanères permettant de remédier aux inconvénients mentionnés ci-dessus.

De façon surprenante, le demandeur a trouvé que l'utilisation de polymères particuliers associés à un ou plusieurs solvants volatils permettait l'obtention d'un stick dont l'application sur les lèvres conduisait à un film ayant des propriétés cosmétiques remarquables. En particulier, le film est brillant, souple, confortable et "sans transfert". De plus, la composition est stable dans le temps et n'exsude pas à température ambiante.

Par stable, on entend une composition qui n'exsude pas à température ambiante pendant au moins 2 moins, voire jusqu'à 9 mois.

L'invention s'applique non seulement aux produits de maquillage des lèvres, comme les rouges à lèvres, les brillants à lèvres et les crayons à lèvres mais aussi aux produits de soin et/ou de traitement de la peau, y compris du cuir chevelu, du visage, du corps et des lèvres comme les produits notamment en stick de protection solaire de la peau du visage ou des lèvres, aux produits de maquillage de la peau, aussi bien du visage que du corps humain, comme les fonds de teints éventuellement coulés en stick ou en coupelle, les produits anticerne, les fards à paupières et les produits de tatouage éphémère, aux produits d'hygiène corporelle comme les déodorants notamment en stick, les shampooings et après- shampooings et aux produits de maquillage des yeux comme les eye-liners, les crayons et les mascaras plus spécialement sous forme de pain, ainsi qu'aux produits de soin des fibres kératiniques comme les cheveux et les sourcils.

De façon plus précise, l'invention a pour objet une composition structurée contenant au moins une phase grasse liquide comprenant au moins un solvant volatil,
- ledit solvant volatil étant choisi parmi les huiles de silicones linéaires ou cycliques ayant une viscosité à température ambiante inférieure à 8 cSt, les huiles volatiles hydrocarbonées ayant de 8 à 16 atomes de carbone, des solvants volatils fluorés, et leurs mélanges à l'exclusion de l'Isopar M,
- la phase grasse liquide étant structurée par au moins un polymère de masse moléculaire moyenne en poids inférieure ou égale à 100000, comportant :
   a) un squelette polymérique, ayant des motifs de répétition hydrocarbonés pourvus d'au moins un hétéroatome, et
   b) au moins une chaîne grasse pendante et/ou terminale éventuellement fonctionnalisée, ayant de 6 à 120 atomes de carbone et étant liées à ces motifs hydrocarbonés,
le polymère étant un polyamide choisi parmi les polymères de formule (I) suivante et leurs mélanges : 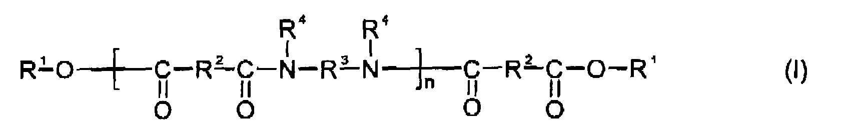 dans laquelle :
- n désigne un nombre de motifs amide tel que le nombre de groupes ester représente de 10 % à 50 % du nombre total des groupes ester et amide
- R¹ est à chaque occurrence indépendamment un groupe alkyle ou alcényle ayant au moins 4 atomes de carbones ;
- R² représente à chaque occurrence indépendamment un groupe hydrocarboné en C₄ à C₄₂ à condition que 50 % des groupes R² représentent un groupe hydrocarboné en C₃₀ à C₄₂ ;
- R³ représente à chaque occurrence indépendamment un groupe organique pourvus d'au moins 2 atomes de carbone, d'atomes hydrogène et optionnellement d'un ou plusieurs atomes d'oxygène ou d'azote ; et
- R⁴ représente à chaque occurrence indépendamment un atome d'hydrogène, un groupe alkyle en C₁ à C₁₀ ou une liaison directe à R³ ou un autre R⁴ de sorte que
- l'atome d'azote auxquels sont liés à la fois R³ et R⁴ fasse partie d'une structure hétérocyclique définie par R⁴-N-R^{3,} avec au moins 50 % des R⁴ représentant un atome d'hydrogène,
la phase grasse liquide et le polymère formant un milieu physiologiquement acceptable,
la composition contenant au moins un polymère liposoluble ou dispersible dans le milieu présentant un poids molécules moyen de 500 à 1 000 000 et choisi parmi les polyalkylènes, les polyméthacrylates, les alkylcelluloses avec un radical alkyl linéaire ou ramifié, saturé ou non en C₁ à C₈, les polymères siliconés, les copolymères de vinylpyrrolidone, les copolymères d'alcène en C₂ à C₃₀, et leurs associations.

Par « au moins une chaîne grasse >, on entend une ou plusieurs chaînes grasses pendantes, une ou plusieurs chaînes grasses terminales, ou l'association de ces chaînes. Avantageusement, la composition de l'invention ne contient pas de résine de silicone à motifs siloxysilicate ou de silice triméthylée, afin de préserver les propriétés de confort de la composition. carboxylique, amine, halogène, dont les groupes fluorés ou perfluorés, ester, siloxane, polysiloxane. En outre, les atomes d'hydrogène d'une ou plusieurs chaînes grasses peuvent être substitués au moins partiellement par des atomes de fluor.

Par « polymère », on entend au sens de l'invention un composé ayant au moins 2 motifs de répétition, de préférence trois motifs identiques.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs hétéroatomes non pendants et se trouvant dans le squelette polymérique. Ces hétéroatomes sont choisis parmi les atomes d'azote, de soufre, de phosphore et leurs associations, et mieux les atomes d'azote, associés éventuellement à un ou plusieurs atome d'oxygène. De préférence, les motifs comportent au moins un atome d'azote en particulier non pendant. Les motifs comportent, en outre, avantageusement un groupe carbonyle.

Ces motifs à hétéroatome sont en particulier des motifs amide formant un squelette du type polyamide, des motifs carbamate et/ou urée formant un squelette polyuréthane, polyurée et/ou polyurée-uréthane. De préférence, ces motifs sont des motifs amide. Avantageusement, les chaînes pendantes sont liées directement à l'un au moins des hétéroatomes du squelette polymérique. Lorsque les motifs répétitifs sont des motifs urée, les chaînes terminales ne sont pas liées au squelette polyurée par un motif uréthane.

Entre, les motifs hydrocarbonés, le polymère peut comprendre des motifs siliconés ou des motifs oxyalkylénés.

En outre, le polymère de la composition de l'invention comprend avantageusement de 40 à 98 % de chaînes grasses par rapport au nombre total des motifs à hétéroatome et des chaînes grasses et mieux de 50 à 95 %. La nature et la proportion des motifs à hétéroatome est fonction de la nature de la phase grasse liquide et est en particulier similaire à la nature de la phase grasse. Ainsi, plus les motifs à hétéroatome sont polaires et en proportion élevée dans le polymère, ce qui correspond à la présence de plusieurs hétéroatomes, plus le polymère a de l'affinité avec les huiles polaires. En revanche, plus les motifs à hétéroatome sont peu polaires voire apolaires ou en proportion faible, plus le polymère a de l'affinité avec les huiles apolaires.

Les chaînes grasses sont liées au squelette polymérique par un groupe de liaison qui peut être une liaison simple, un groupe urée, uréthane, ester, éther, amine, thioéther, thioester, thiourée, thiouréthane, ou leurs associations. De préférence, le groupe de liaison est le groupe ester.

L'invention a aussi pour objet une composition structurée contenant au moins une phase grasse liquide comprenant au moins un solvant volatil, la phase grasse liquide étant structurée par au moins un polyamide de masse moléculaire moyenne en poids inférieur ou égal à 100 000, comportant a) un squelette polymérique, ayant des motifs répétitifs amide, et b) éventuellement au moins une chaîne grasse pendante et/ou terminale éventuellement fonctionnalisée, ayant de 6 à 120 atomes de carbone et étant liée à ces motifs amide, la phase grasse liquide et le polymère formant un milieu physiologiquement acceptable.

De préférence, les chaînes grasses pendantes sont liées à l'un au moins des atomes d'azote des motifs amide.

En particulier, les chaînes grasses de ce polyamide représentent de 40 à 98% du nombre total des motifs amide et des chaînes grasses, et mieux de 50 à 95% Comme polymères structurant préférés utilisables dans l'invention, on peut citer les polyamides ramifiés par des chaînes grasses pendantes et/ou des chaînes grasses terminales ayant de 6 à 120 atomes de carbone, et notamment de 12 à 120 atomes de carbone, et mieux de 12 à 68 atomes de carbone, les chaînes grasses terminales étant liées au squelette polyamide par des groupes ester.

Ces polymères sont de préférence des polymères résultant d'une polycondensation entre un diacide carboxylique à au moins 32 atomes de carbone (ayant notamment de 32 à 44 atomes de carbone) avec une diamine ayant au moins 2 atomes de carbone (ayant notamment de 2 à 36 atomes de carbone). Le diacide est de préférence un dimère d'acide gras ayant au moins 16 atomes de carbone comme l'acide oléïque, linoléïque, linolénïque. La diamine est de préférence l'éthylène diamine, l'hexylène diamine, l'hexaméthylène diamine, le phénylène diamine, l'éthylène triamine et encore mieux l'éthylène diamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple à 18 atomes de carbone.

Ces polymères sont plus spécialement ceux décrits dans le document US-A-5783657 de la société Union Camp. Chacun de ces polymères satisfait notamment à la formule (1) suivante : 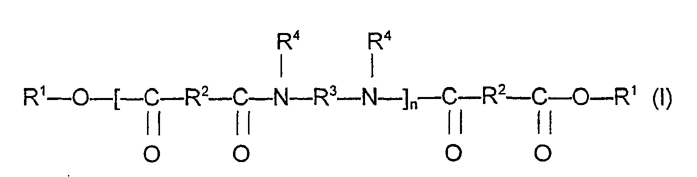 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone, et notamment de 4 à 24 atomes de carbone ; R² représente à chaque occurrence indépendamment un groupe hydrocarboné en C₄ à C₄₂ à condition que 50 % des groupes R² représentent un groupe hydrocarboné en C₃. à C₄₂ ; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

Dans le cas particulier de la formule (I), les chaînes grasses terminales éventuellement fonctionnalisées au sens de l'invention sont les chaînes terminales liées au dernier hétéroatome, ici azote, du squelette polyamide.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5 et mieux de 3 à 5. De préférence, R¹ est un groupe alkyle en C₁₂ à C₂₂ et de préférence en C₁₆ à C₂₂. Avantageusement, R peut être un groupe hydrocarboné (alkylène) en C₁₀ à C₄₂. De préférence, 50 % au moins et mieux 75 % des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C_{12'}

Les groupes hydrocarbonés peuvent être des groupes linéaires, cycliques ou ramifiés, saturés ou insaturés. Par ailleurs, les groupes alkyles et alkylènes peuvent être des groupes linéaires ou ramifiés, saturés ou non.

De façon avantageuse, le polymère de la composition de l'invention comprend une masse moléculaire moyenne en poids allant 1000 à 30 000, de préférence allant de 2 000 à 20 000, et mieux de 2 000 à 10 000.

Selon l'invention, la structuration de la phase grasse liquide est obtenue à l'aide d'un ou plusieurs polymères de formule (I). En général, les polymères de formule (I) se présentent sous forme de mélanges de polymères, ces mélanges pouvant en outre contenir un produit de synthèse (correspondant à un composé de formule (I) avec n valant 0), c'est-à-dire un diester.

A titre d'exemple de polymères structurant utilisables dans la composition selon l'invention, on peut citer les produits commerciaux vendu par la société Bush Boake Allen sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en C₃₆ condensé sur l'éthylène diamine, de masse moléculaire moyenne d'environ 6000.

Les groupes ester terminaux résultent de l'estérification des terminaisons d'acide restantes par l'alcool cétylique, stéarylique ou leurs mélanges (appelés aussi alcool cétylstéarylique).

Comme polymère structurant utilisable dans l'invention, on peut encore citer les résines polyamides résultant de la condensation d'un acide di-carboxylique aliphatique et d'un diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid^{®} par les sociétés General Mills, Inc. et Henkel Corp. (Versamid 930, 744 ou 1655) ou par la société Olin Mathieson Chemical Corp., sous la marque Onamid^{®} notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid^{®} 930 ou 744.

On peut aussi utiles les polyamides vendus par la société Union Camp Corp. sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Les polymères structurant de la composition de l'invention ont avantageusement une température de ramollissement supérieur à 65°C et notamment supérieur à 70°C et pouvant aller jusqu'à 190°C. De préférence, il présente une température de ramollissement allant de 80 à 130°C et mieux de 80 à 105°C. Ces polymères sont en particulier des polymères non cireux et/ou non cristallins. Cette température de ramollissement est plus basse que celle des polymères structurant connus, ce qui facilite la mise en oeuvre des polymères objet de l'invention et limite les détériorations de la phase grasse liquide.

Les polymères à chaîne(s) grasse(s), objet de l'invention, présentent du fait de leur chaîne grasse, une bonne solubilité dans les huiles (à savoir composés liquides, non miscibles à l'eau) et donc conduisent à des compositions macroscopiquement homogènes même avec un taux élevé (au moins 25%) de polymère, contrairement aux polymères exempts de chaîne grasse.

Avantageusement, le polymère est associé à au moins un composé amphiphile liquide et non volatile à température ambiante, de valeur de balance hydrophile/lipophile (HLB) inférieure à 12 et notamment allant de 1 à 8 et de préférence de 1 à 5. Selon l'invention, on peut utiliser un ou plusieurs composés amphiphiles. Ces composés amphiphiles ont pour but de renforcer les propriétés structurantes du polymère à hétéroatome, de faciliter la mise en oeuvre du polymère et d'améliorer la capacité à déposer du stick.

Selon l'invention, la composition peut avoir une dureté allant de 20 à 800 g et mieux de 20 à 900 g, notamment de 20 à 600g et, par exemple, de 150 à 450 g. Cette dureté peut être mesurée selon une méthode de pénétration d'une sonde dans ladite composition et en particulier à l'aide d'un analyseur de texture (par exempleTA-XT2i : de chez Rhéo) équipé d'un cylindre en ébonite de 25 mm de haut et 8 mm de diamètre. La mesure de dureté est effectuée à 20°C au centre de 5 échantillons de la dite composition. Le cylindre est introduit dans chaque échantillon de composition à une pré-vitesse de 2mm/s puis à une vitesse de 0,5 mm/s et enfin à une post-vitesse de 2mm/s, le déplacement total étant de 1 mm. La valeur relevée de la dureté est celle du pic maximum. L'erreur de mesure est de ± 50g.

La dureté peut aussi être mesurée par la méthode dite du fil à couper le beurre, qui consiste à couper un bâton de rouge à lèvres de 8,1 mm et à mesurer la dureté à 20°C, au moyen d'un dynamomètre DFGHS 2 de la société Indelco-Chatillon se déplaçant à une vitesse de 100mm/minute. Elle est exprimée comme la force de cisaillement (exprimée en gramme) nécessaire pour couper un stick dans ces conditions. Selon cette méthode la dureté d'une composition en stick selon l'invention va de 30 à 150 g, de préférence de 30 à 120 g et, par exemple, de 30 à 50 g.

La dureté de la composition selon l'invention est telle que la composition est autoportée et peut se déliter aisément pour former un dépôt satisfaisant sur la peau et/ou les lèvres et/ou les phanères. En outre, avec cette dureté, la composition de l'invention résiste bien aux chocs.

Selon l'invention, la composition sous forme de stick a le comportement d'un solide élastique déformable et souple, conférant à l'application une douceur élastique remarquable. Les compositions en stick de l'art antérieur n'ont pas cette propriété d'élasticité et de souplesse.

Le ou les composés amphiphiles utilisables dans la composition de l'invention comprennent une partie lipophile liée à une partie polaire, la partie lipophile comportant une chaîne carbonée ayant au moins 8 atomes de carbone notamment, de 18 à 32 atomes de carbone et mieux de 18 à 28 atomes de carbone. De préférence, la partie polaire de ce ou ces composés amphiphiles est le reste d'un composé choisi parmi les alcools et les polyols ayant de 1 à 12 groupements hydroxyle, les polyoxyalkylènes comportant au moins 2 motifs oxyalkylénés et ayant de 0 à 20 motifs oxypropylénés et/ou de 0 à 20 motifs oxyéthylénés. En particulier, le composé amphiphile est un ester choisi parmi les hydroxystéarates, les oléates, les iso-stéarates du glycérol, du sorbitan ou du méthylglucose ou encore les alcools gras ramifiés en C₁₂ à C₂₆ comme l'octyldodécanol et leurs mélanges. Parmi ces esters, on préfère les monoesters et les mélanges de mono- et de di-esters.

Le taux de composé amphiphile et celui du polymère à hétéroatome sont choisis selon la dureté de gel désirée et en fonction de l'application particulière envisagée. Les quantités respectives de polymère et de composé amphiphile doivent être telles qu'elles permettent l'obtention d'un stick délitable. En pratique, la quantité de polymère représente de 0,5 à 80 % du poids total de la composition et mieux de 5 à 40 %. La quantité de composé amphiphile représente en pratique de 0,1 % à 35 % du poids total de la composition et mieux de 1 % à 15 %, s'il est présent.

Avantageusement, la phase grasse liquide de la composition contient plus de 30 % et mieux plus de 40. % d'huile(s) liquide(s) ayant une affinité avec les motifs à hétéroatome (structure chimique proche) et mieux de 50 à 100 %. En particulier, la phase grasse liquide structurée par un squelette de type polyamide contient une quantité majoritaire, à savoir supérieure à 30 % et mieux à 40 % du poids total de la phase grasse liquide et mieux de 50 à 100 %, d'huile ou mélange d'huiles liquides apolaires, et plus spécialement d'huile(s) hydrocarbonée(s).

Par « huile hydrocarbonée », on entend au sens de l'invention des huiles comprenant majoritairement des atomes de carbone et des atomes d'azote, ces huiles comportant éventuellement un groupe ester, éther, acide ou alcool.

Pour une phase grasse liquide structurée par un polymère comportant un squelette en partie siliconée, cette phase grasse contient de préférence plus de 30 % et mieux plus de 40 % du poids total de la phase grasse liquide et mieux de 50 à 100 %, d'huile ou mélange d'huiles liquides siliconées, par rapport au poids total de la phase grasse liquide.

Pour une phase grasse liquide structurée par un polymère apolaire du type hydrocarboné, cette phase grasse contient avantageusement plus de 30 % et mieux plus de 40 % en poids et mieux de 50 à 100 %, d'huile ou mélange d'huiles apolaires liquides, notamment hydrocarbonées, par rapport au poids total de la phase grasse liquide.

En particulier, les huiles polaires de l'invention sont :
- les huiles végétales hydrocarbonées à forte teneur en triglycérides constitués d'esters d'acides gras et de glycérol dont les acides gras peuvent avoir des longueurs de chaînes variées de C₄ à C_{24'} ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de coton, de luzerne, de pavot, de potimarron, de sésame, de courge, de colza, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse ou esters de synthèse de formule R₅COOR₆ dans laquelle R₅ représente le reste d'un acide gras linéaire ou ramifié comportant de 1 à 40 atomes de carbone et R₆ représente une chaîne hydrocarbonée notamment ramifiée contenant de 1 à 40 atomes de carbone à condition que R₅ + R₆ soit ≥ 10, comme par exemple l'huile de Purcellin (octanoate de cétostéaryle), l'isononanoate d'isononyle, le benzoate d'alcool en C₁₂ à C₁₅, le myristate d'isopropyle, le palmitate d'éthyl 2-hexyle, l'isostéarate d'isostéarate, des octanoates, décanoates ou ricinoléates d'alcools ou de polyalcools ; les esters hydroxylés comme le lactate d'isostéaryle, le malate de di-isostéaryle ; et les esters du pentaérythritol ;
- les éthers de synthèse ayant de 10 à 40 atomes de carbone ;
- les alcools gras en C₈ à C₂₆ comme l'alcool oléique ;
- les acides gras en C₈ à C₂₆ comme l'acide oléique ;
- leurs mélanges.

Les huiles apolaires selon l'invention sont en particulier les huiles siliconées telles que les polydiméthylsiloxanes (PDMS) volatils ou non, linéaires ou cycliques, liquides à température ambiante ; les polydiméthylsiloxanes comportant des groupements alkyle, alcoxy ou phényle, pendant et/ou en bout de chaîne siliconée, groupements ayant de 2 à 24 atomes de carbone ; les silicones phénylées comme les phényl triméthicones, les phényl diméthicones, les phényl triméthylsiloxy diphénylsiloxanes, des diphényl diméthicones, les diphényl méthyldiphényl trisiloxanes, les 2-phényléthyl triméthylsiloxysilicates ; les hydrocarbures linéaires ou ramifiés d'origine synthétique ou minérale comme les huiles de paraffine, volatiles ou non volatiles, et ses dérivés, la vaseline, la lanoline liquide, les polydécènes, le polyisobutène hydrogéné tel que le parléam, le squalane ; et leurs mélanges. De préférence, les huiles structurées, et plus spécialement celles structurées par les polyamides et en particulier ceux de formules (I) ou les polyuréthanes ou les polyurées ou les polyurée-uréthanes, sont des huiles apolaires et plus spécialement une huile ou un mélange d'huiles du type hydrocarboné d'origine minérale ou synthétique, choisies en particulier parmi les hydrocarbures notamment les alcanes comme l'huile de parléam, les isoparaffines comme l'isododécane et le squalane et leurs mélanges. Avantageusement, ces huiles sont associées à une pou plusieurs huiles de silicones phénylées.

De préférence, la phase grasse liquide contient, au moins une huile non volatile choisie en particulier parmi les huiles hydrocarbonées d'origine minérale, végétale ou synthétique, les esters ou éthers de synthèse, les huiles de silicone et leurs mélanges.

La phase grasse liquide totale représente, en pratique, de 3 à 99,5 % et, notamment, de 5 à 99,5 % du poids total de la composition, de préférence de 20 à75%.

La phase grasse liquide de la composition selon l'invention contient, en outre, au moins un solvant volatil, à savoir un ou plusieurs solvants volatils.

Par "solvant volatil", on entend au sens de l'invention tout milieu non aqueux susceptible de s'évaporer au contact de la peau ou des lèvres en moins d'une heure, à température ambiante et pression atmosphérique. Le ou les solvants volatils de l'invention sont des solvants organiques et notamment des huiles cosmétiques volatiles, liquides à température ambiante, ayant une pression de vapeur non nulle, à température ambiante et pression atmosphérique, allant en particulier de 10⁻³ à 300 mm de Hg (0,13 Pa à 40.000 Pa) et de préférence supérieur à 0,03 mm de Hg (3,9 Pa).

De préférence, les solvants volatils de l'invention sont des huiles cosmétiques choisies parmi les huiles n'ayant pas de point éclair, les huiles ayant un point éclair allant de 40°C à 100°C, et leurs mélanges, en vue de faciliter leur mise en oeuvre. En outre, ils présentent avantageusement une température d'ébullition à pression atmosphérique inférieure à 220°C et mieux inférieure à 210°C, notamment allant de 110 à 210°C.

Selon l'invention, ces solvants volatils facilitent, notamment, l'application de la composition sur la peau, les lèvres ou les phanères. Ces solvants peuvent être des solvants hydrocarbonés, des solvants siliconés comportant éventuellement des groupements alkyle ou alkoxy pendants ou en bout de chaîne siliconée ou un mélange de ces solvants. De préférence, ces solvants ne sont pas des monoalcools à au moins 7 atomes de carbone.

Comme solvant volatil utilisable dans l'invention, on peut citer les huiles de silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Comme autre solvant volatil utilisable dans l'invention, on peut citer les huiles volatiles hydrocarbonées ayant de 8 à 16 atomes de carbone et leurs mélanges et notamment les alcanes ramifiés en C₈-C₁₆ comme les iso-alcanes (appelées aussi isoparaffines) en C₈-C₁₆, et notamment en C₈-C₁₃, l'isodécane, l'isohexadécane et par exemple les huiles vendues sous les noms commerciaux d'Isopars L, E, H, G ou de Permetyls, les esters ramifiés en C₈-C₁₆ comme le néopentanoate d'iso-hexyle et leurs mélanges. De préférence, le solvant volatil est choisi parmi les huiles volatiles hydrocarbonées ayant de 8 à 16 atomes de carbone et leurs mélanges, à l'exclusion de l'Isopar M.

On peut aussi utiliser des solvants volatils fluorés.

De préférence, on utilise l'isododécane (Permetyls 99 A), les isoparaffines en C₈-C₁₆ (Isopars L,E,H) , leurs mélanges, éventuellement associés au décaméthyl tétrasiloxane ou au cyclopentasiloxane.

Ces huiles volatiles représentent notamment un taux massique de 3 à 99,5 % par rapport au poids total de la composition, et notamment de 5 à 97,5 %, de préférence de 10 à 75 % et mieux de 15 à 45 %. De façon générale, la quantité de solvant volatil est utilisée en une quantité suffisante pour obtenir des propriétés de sans transfert. Cette quantité sera adaptée par l'homme du métier en fonction de l'intensité des propriétés de sans transfert recherchée.

La composition de l'invention peut comprendre, en outre, tout additif usuellement utilisé dans le domaine concerné, choisi notamment parmi les matières colorantes, les antioxydants, les huiles essentielles, les conservateurs, les parfums, les charges, les cires, les produits pâteux à température ambiante, les neutralisants, les polymères liposolubles ou dispersibles dans le milieu, les actifs cosmétiques ou dermatologiques ayant une action biologique sur la peau comme par exemple des émollients, des hydratants, des vitamines, des acides gras essentiels, des filtres solaires, les dispersants comme l'acide poly(12-hydroxystéarique), et leurs mélanges. Ces additifs peuvent être présents dans la composition à raison de 0 à 20% (notamment de 0,01 à 20 %) du poids total de la composition et mieux de 0,01 à 10%. Avantageusement, la composition contient au moins un actif cosmétique ou dermatologique, ayant une action biologique.

La composition de l'invention peut, en outre contenir comme additif une phase aqueuse contenant de l'eau éventuellement épaissie ou gélifiée par un épaississant ou un gélifiant de phase aqueuse et éventuellement des composés miscibles à l'eau.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut se présenter sous la forme d'une composition teintée dermatologique ou de soin des matières kératiniques comme la peau, les lèvres et/ou les phanères, sous forme d'une composition de protection solaire ou d'hygiène corporelle notamment sous forme de produit déodorant ou démaquillant sous forme de stick. Elle peut notamment être utilisée comme base de soin pour la peau, les phanères ou les lèvres (baumes à lèvres, protégeant les lèvres du froid et/ou du soleil et/ou du vent, crème de soin pour la peau, les ongles ou les cheveux).

La composition de l'invention peut également se présenter sous la forme d'un produit de maquillage, notamment coloré, de la peau, en particulier un fond de teint, présentant éventuellement des propriétés de soin ou de traitement, un blush, un fard à joues ou à paupières, un produit anti-cerne, un eye-liner, un produit de maquillage du corps ; de maquillage des lèvres comme un rouge à lèvres, présentant éventuellement des propriétés de soin ou de traitement ; de maquillage des phanères comme les ongles, les cils en particulier sous forme d'un mascara pain, les sourcils et les cheveux notamment sous forme de crayon. La composition de l'invention est avantageusement un produit de maquillage.

Bien entendu la composition de l'invention doit être cosmétiquement ou dermatologiquement acceptable, à savoir contenir un milieu physiologiquement acceptable non toxique et susceptible d'être appliquée sur la peau, les phanères ou les lèvres d'êtres humains. Par cosmétiquement acceptable, on entend au sens de l'invention une composition d'aspect, d'odeur, de goût et de toucher agréables.

Avantageusement, la composition contient au moins un actif cosmétique et/ou un actif dermatologique et/ou au moins une matière colorante. Grâce à l'association d'au moins un solvant volatil et d'au moins un polymère de masse moléculaire moyenne en poids allant de 1000 à 30 000, tels que défini précédemment, on obtient un piégeage des actifs et des matières colorantes présents dans la composition, permettant de les maintenir la où ils ont été appliqués, à savoir les lèvres, la peau ou les phanères comme les fibres kératiniques, après évaporation du ou des solvants volatils, et de limiter leur transfert ou redépôt sur un support différent de celui sur lequel ils ont été appliqués.

La matière colorante selon l'invention peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 50 % du poids total de la composition, de préférence de 5 à 30 %, si elle est présente et mieux de 8 à 20 %.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine, le rocou. Ils peuvent représenter de 0,1 à 20 % du poids de la compositions et mieux de 0,1 à 6 %.

Les pigments peuvent être blancs ou colorés, minéraux et/ou organiques, enrobés ou non. On peut citer, parmi les pigments minéraux, le dioxyde de titane ou de zinc, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les pigments peuvent représenter de 0,1 à 50 % et mieux de 2 à 30 % du poids total de la composition, s'ils sont présents

Les pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0,1 à 20 % du poids total de la composition et mieux de 0,1 à 15 %, s'ils sont présents.

La composition peut éventuellement contenir une ou plusieurs cires pour améliorer la structuration sous forme de stick, bien que cette forme rigide puisse être obtenue en l'absence de cire. Une cire, au sens de la présente invention, est un composé gras lipophile, solide à température ambiante (25°C), à changement d'état solide/liquide réversible, ayant une température de fusion supérieure à 45°C et mieux à 55°C pouvant aller jusqu'à 200°C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange. C'est cette recristallisation dans le mélange qui est responsable de la diminution de la brillance dudit mélange. Aussi, avantageusement la composition contient peu ou pas de cire, et notamment moins de 5 % de cire.

Les cires, au sens de la demande, sont celles généralement utilisées dans les domaines cosmétique et dermatologique ; elles sont notamment d'origine naturelle comme la cire d'abeilles, la cire de Carnauba, de Candellila, d'Ouricoury, du Japon, de fibres de liège ou de canne à sucre, les cires de paraffine, de lignite, les cires microcristallines, la cire de lanoline, la cire de Montan, les ozokérites, les huiles hydrogénées comme l'huile de jojoba hydrogénée, mais aussi d'origine synthétique comme les cires de polyéthylène issues de la polymérisation de l'éthylène, les cires obtenues par synthèse de Fischer-Tropsch, les esters d'acides gras et les glycérides concrets à 45°C, les cires de silicone comme les alkyle, alcoxy et/ou esters de poly(di)méthylsiloxane solide à 45°C.

Les valeurs de température sont notamment celles de la méthode connue « Dynamic Scannig Calorimetry ».

La composition de l'invention contient au moins un polymère liposoluble ou dispersible dans le milieu présentant notamment un poids moléculaire moyen de 500 à 1 000 000 et mieux de 5 000 à 15 000. Ce ou ces polymères liposolubles contribuent notamment à augmenter la viscosité et/ou améliorer la tenue du film. Ces polymères liposolubles présentent avantageusement une température de ramollissement au plus égale à 30° C.

A titre d'exemple de polymères liposolubles utilisables dans l'invention, on peut citer : les polyalkylènes, notamment le polybutène, les poly(méth)acrylates, les alkylcelluloses avec un radical alkyl linéaire ou ramifié, saturé ou non en C₁ à C₈ comme l'éthylcellulose et la propylcellulose, les polymères siliconés compatibles avec la phase grasse ainsi que les copolymères de la vinylpyrrolidone (VP) et leurs mélanges.

De préférence, on utilise les copolymères de la vinylpyrrolidone, les copolymères d'alcène en C₂ à C₃₀ et mieux en C₃ à C₂₂, et leurs associations. A titre d'exemple de copolymère de VP utilisable dans l'invention, on peut citer le copolymère de VP/acétate vinyle, VP/méthacrylate d'éthyle, la polyvinylpyrrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

De façon préférentielle, non seulement pour les propriétés de tenue mais aussi de toucher et de consistance du film, on utilise le copolymère PVP/hexadécène ayant un poids moléculaire moyen de 7000 à 7500 ou encore le PVP/eicosène ayant un poids moléculaire moyen de 8000 à 9000.

Les polymères liposolubles ou dispersibles de la composition de l'invention sont avantageusement utilisés dans une quantité de 0,01 % à 20 % (en matière active) du poids total de la composition et mieux de 1% à 10%.

La composition selon l'invention contient, en outre, avantageusement au moins un composé gras pâteux à température ambiante. Par « corps gras pâteux » au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L' homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.
Selon l'invention, on utilise un ou plusieurs corps gras pâteux. De préférence, ces corps gras sont des composés hydrocarbonés, éventuellement de type polymérique ; ils peuvent également être choisis parmi les composés siliconés et/ou fluorés ; il peut aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le « THIXINR » de Rheox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le ou les corps gras pâteux peuvent être présents à raison de 0,1 à 60% en poids, par rapport au poids total de la composition, de préférence à raison de 1-45% en poids et encore plus préférentiellement à raison de 2-30% en poids, dans la composition, s'ils sont présents.

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramollissement, à y ajouter le ou les composés amphiphiles, les matières colorantes et les additifs puis à mélanger le tout jusqu'à l'obtention d'une solution claire, transparente. On ajoute alors, au mélange obtenu, après abaissement de la température le ou les solvants volatils. Le mélange homogène obtenu peut alors être coulé dans un moule approprié comme un moule de rouge à lèvres ou directement dans les articles de conditionnement (boîtier ou coupelle notamment).

L'invention a encore pour objet une composition de rouge à lèvres en stick contenant au moins une phase grasse liquide continue comprenant au moins un solvant volatil, la phase grasse liquide étant structurée par au moins un polymère non cireux conférant à la composition l'aspect d'un solide déformable, élastique, de dureté allant de 30 à 50 g, en l'absence de cire.

Avantageusement cette composition de rouge à lèvres en stick contient un additif choisi parmi les composés gras pâteux à température ambiante, les polymères liposolubles et leurs mélanges, tels que définis précédemment. Le polymère non cireux est de préférence un polymère dont le squelette comporte des motifs hydrocarbonés à hétèroatome, tel que défini précédemment.

L'invention a encore pour objet un procédé cosmétique de soin, de maquillage ou de traitement des matières kératiniques des êtres humains et notamment de la peau, des lèvres et des phanères, comprenant application sur les matières kératiniques de la composition notamment cosmétique telle que définie ci-dessus.

L'invention a aussi pour objet l'utilisation de l'association d'au moins un solvant volatil choisi parmi les huiles de silicone linéaires ou cycliques ayant une viscosité à température ambiante inférieure à 8 cSt, les huiles volatiles hydrocarbonées ayant de 8 à 16 atomes de carbone et leurs mélanges, des solvants volatils fluorés, et leurs mélanges, d'au moins un polymère de masse moléculaire moyenne en poids allant de 1000 à 30 000, comportant a) un squelette polymérique, ayant des motifs de répétition hydrocarbonés pourvus d'au moins un hétéroatome, et b) éventuellement au moins une chaîne grasse pendante et/ou terminale éventuellement fonctionnalisée, le polymère étant un polyamide choisi parmi les polymères de formule (I) suivante et leurs mélanges : 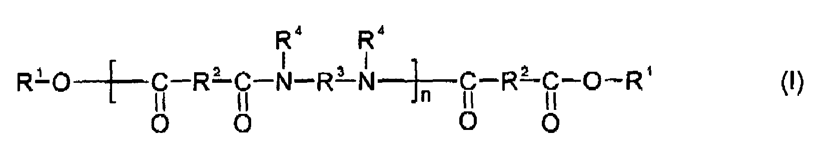 dans laquelle :
- n désigne un nombre de motifs amide tel que le nombre de groupes ester représente de 10 % à 50 % du nombre total des groupes ester et amide
- R¹ est à chaque occurrence indépendamment un groupe alkyle ou alcényle ayant au moins 4 atomes de carbones ;
- R² représente à chaque occurrence indépendamment un groupe hydrocarboné en C₄ à C₄₂ à condition que 50 % des groupes R² représentent un groupe hydrocarboné en C₃₀ à C₄₂ ; 1
- R³ représente à chaque occurrence indépendamment un groupe organique pourvus d'au moins 2 atomes de carbone, d'atomes hydrogène et optionnellement d'un ou plusieurs atomes d'oxygène ou d'azote ; et
- R⁴ représente à chaque occurrence indépendamment un atome d'hydrogène, un groupe alkyle en C₁ à C₁₀ ou une liaison directe à R³ ou un autre R⁴ de sorte que l'atome d'azote auxquels sont liés à la fois R³ et R⁴ fasse partie d'une structure hétérocyclique définie par R⁴-N-R³, avec au moins 50 % des R⁴ représentant un atome d'hydrogène, et
d'au moins un polymère liposoluble ou dispersible dans le milieu présentant un poids moléculaire moyen de 500 à 1 000 000 et choisi parmi les polyalkylènes, les polyméthacrylates, les alkylcelluloses avec un radical alkyl linéaire ou ramifié, saturé ou non en C₁ à C₈, les polymères siliconés, les copolymères de vinylpyrrolidone, les polymères d'alcène en C₂ à C₃₀, et leurs associations, dans une composition cosmétique ou pour la fabrication d'une composition physiologiquement acceptable, pour diminuer le transfert et/ou le dépôt de traces d'un film de ladite composition, appliqué sur les matières kératiniques, sur un support mis au contact dudit film et/ou augmenter la tenue dudit film. Ce film est, en outre, brillant et/ou confortable.

L'invention est illustrée plus en détail dans les exemples suivants. Les pourcentages sont donnés en pourcentage massique.

### Phase B

| | |
|---|---|
| . Pigments | 10 % |
| . Iso-paraffine hydrogénée | 5 % |
| . Lanoline liquide | 5 % |
| . Acide pofy(12-hydroxystéarique) | 2 % |

### Phase C

| | |
|---|---|
| .Isododécane | 25 % |
| . Décaméthyl tétrasiloxane | 10 % |

La phase pigmentaire (B) est broyée à l'aide d'un broyeur tri-cylindre et introduite dans la phase huileuse A préalablement chauffée à 100°C jusqu'à homogénéisation complète du mélange. On ajoute ensuite la phase volatile C dans le mélange précédant ramené à 85°C. L'ensemble est laissé en contact pendant 10 min puis coulé dans des moules de rouge à lèvres.

Le rouge à lèvres obtenu dépose un film brillant et non-transfert. Ce rouge à lèvres a été jugé, par des personnes testrices, de tenue égale et de propriété non-transfert et non-migrante ou équivalentes et à celles d'un rouge à lèvres non-transfert de l'art antérieur tel que décrit dans l'exemple 1 du document EP-A-847752, mais plus brillant que celui de l'art antérieur. Ce rouge à lèvres connu contenait :

| | |
|---|---|
| . PDMS (100 Cst) | 8 % |
| Polyisobutène hydrogéné | 18 % |
| . Propionate d'arachidyle | 7,5 % |
| . Cire de polyéthylène | 16,5 % |
| . Pigments/nacres | 11 % |
| . Isododécane | qsp 100% |

### Exemple 2 : Rouge à lèvres

### Phase A

| | |
|---|---|
| . Uniclear 100 | 18 % |
| . Huile de ricin | 8 % |
| . Iso-paraffine hydrogénée | 5 % |
| . Isononanoate d' isononyle | 5 % |
| . Phényl triméthylsiloxy trisiloxane | 8 % |
| . Copolymère vinylpyrrolidone / 1-eicosène | 2 % |

### Phase B

| | |
|---|---|
| . Pigments | 10 % |
| . Iso-paraffine hydrogénée | 5 % |
| . Lanoline liquide | 5 % |
| . Acide poly(12-hydroxystéarique) | 2 % |

### Phase C

| | |
|---|---|
| . Isododécane | 27 % |
| . Décaméthyl tétrasiloxane | 5 % |

La phase pigmentaire (B) est broyée à l'aide d'un broyeur tri-cylindre et introduite dans la phase huileuse A préalablement chauffée à 100°C jusqu'à homogénéisation complète du mélange. On ajoute ensuite la phase volatile C dans le mélange précédant ramené à 85°C. L'ensemble est laissé en contact pendant 10 min puis coulé dans des moules de rouge à lèvres.

Le rouge à lèvres obtenu dépose un film brillant et non-transfert. Ce rouge à lèvres a été jugé, par un panel de testeurs, de tenue égale et propriétés de non-transfert et non-migration équivalentes à celles d'un rouge à lèvres non-transfert de l'art antérieur conforme à celui de l'exemple 1 du document EP-A-847752, mais plus brillant que celui de l'art antérieur.

## Revendications

1. Composition cosmétique structurée
- contenant au moins une phase grasse liquide comprenant au moins un solvant volatils,
- ledit solvant volatil étant choisi parmi les huiles de silicones linéaires ou cycliques ayant une viscosité à température ambiante inférieure à 8 cSt, les huiles volatiles hydrocarbonées ayant de 8 à 16 atomes de carbone, des solvants volatils fluorés, et leurs mélanges à l'exclusion de l'Isopar M,
- la phase grasse liquide étant structurée par au moins un polymère de masse moléculaire moyenne en poids inférieure ou égale à 100000, comportant :
a) un squelette polymérique, ayant des motifs de répétition hydrocarbonés pourvus d'au moins un hétéroatome, et
b) au moins une chaîne grasse pendante et/ou terminale éventuellement fonctionnalisée, ayant de 6 à 120 atomes de carbone et étant liées à ces motifs hydrocarbonés,
le polymère étant un polyamide choisi parmi les polymères de formule (I) suivante et leurs mélanges : 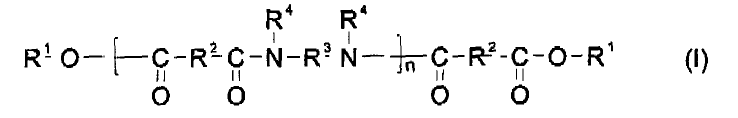 dans laquelle:
- n désigne un nombre de motifs amide tel que le nombre de groupes ester représente de 10 % à 50 % du nombre total des groupes ester et amide ;
- R¹ est à chaque occurrence indépendamment un groupe alkyle ou alcényle ayant au moins 4 atomes de carbone ;
- R² représente à chaque occurrence indépendamment un groupe hydrocarboné en C₄ à C₄₂ à condition que 50 % des groupes R² représentent un groupe hydrocarboné en C₃₀ à C₄₂ ;
- R³ représente à chaque occurrence indépendamment un groupe organique pourvus d'au moins 2 atomes de carbone, d'atomes hydrogène et optionnellement d'un ou plusieurs atomes d'oxygène ou d'azote ; et
- R⁴ représente à chaque occurrence indépendamment un atome d'hydrogène, un groupe alkyle en C₁ à C₁₀ ou une liaison directe à R³ ou un autre R⁴ de sorte que l'atome d'azote auxquels sont liés à la fois R³ et R⁴ fasse partie d'une structure hétérocyclique définie par R⁴-N-R³, avec au moins 50 % des R⁴ représentant un atome d'hydrogène,
la phase grasse liquide et le polymère formant un milieu physiologiquement acceptable, la composition contenant au moins un polymère liposoluble ou dispersible dans le milieu, présentant un poids moléculaire moyen de 500 à 1 000000 et choisi parmi les polyalkylènes, les polyméthacrylates, les alkylcelluloses avec un radical alkyl linéaire ou ramifiés, saturé ou non en C₁ à C₈, les polymères siliconés, les copolymères de vinylpyrrolidone, les copolymères d'alcène en C₂ à C₃₀, et leurs associations.

2. Composition selon la revendication précédente, **caractérisée en ce que** les chaînes grasses représentent de 40 à 98 °/a du nombre total des motifs à hétéroatome et des chaînes grasses.

3. Composition selon la revendication 1 ou 2, **caractérisée en ce que** les chaînes grasses représentent de 50 à 95 % du nombre total des motifs à hétéroatome et des chaînes grasses.

4. Composition selon l'une des revendications précédentes, **caractérisée en ce que** les chaînes grasses pendantes sont liées directement à l'un au moins desdits hétéroatomes.

5. Composition selon l'une des revendications précédentes, **caractérisée en ce que** la masse molaire moyenne en poids va de 1000 à 30000, de préférence de 2000 à 20000 et mieux de 2 000 à 10 000.

6. Composition selon l'une des revendications précédentes, **caractérisée en ce que** les chaînes grasses terminales sont liées au squelette par des groupes ester.

7. Composition selon l'une des revendications précédentes, **caractérisée en ce que** les chaînes grasses ont de 12 à 120 atomes de carbone, et mieux de 12 à 68 atomes de carbone.

8. Composition selon l'une des revendications précédentes, **caractérisée en ce que** R' est un groupe alkyle en C₁₂ à C₂₂.

9. Composition selon l'une des revendications précédentes, **caractérisée en ce que** R2 sont des groupes ayant de 30 à 42 atomes de carbone.

10. Composition selon l'une des revendications précédentes, **caractérisée en ce que** le polymère représente de 0,5 à 80 % du poids total de la composition et mieux de 5 à 40 %.

11. Composition selon l'une des revendications précédentes, dans laquelle le solvant volatil est choisi parmi les huiles n'ayant pas de point éclair, les huiles ayant un point éclair allant de 40 à 100°C, et leurs mélanges.

12. Composition selon l'une des revendications précédentes, **caractérisée en ce que** le solvant volatil est choisis parmi les alcanes ramifiés en C₈-C₁₆, les esters ramifiés en C₈-C₁₆ et leurs mélanges.

13. Composition selon l'une des revendications précédentes, **caractérisée en ce que** le solvant volatil est choisi parmi les isoparaffines en C₈-C₁₆, et notamment en C₈-C₁₃, l'isododécane et leurs mélanges.

14. Composition selon l'une des revendications précédentes, **caractérisée en ce que** le solvant volatil représente un taux massique de 3 à 99,5 %, de préférence de 10 à 75 % et mieux de 15 à 45 %.

15. Composition selon l'une des revendications précédentes, **caractérisée en ce que** la phase grasse liquide contient, en outre, au moins une huile non volatile.

16. Composition selon l'une des revendications précédentes, **caractérisée en ce que** la phase, grasse liquide contient, en outre, au moins une huile non volatile choisie parmi les huiles hydrocarbonées d'origine minérale, végétale ou synthétique, les esters ou éthers de synthèse, les huiles de silicone et leurs mélanges.

17. Composition selon l'une des revendications précédentes, **caractérisée en ce que** la phase grasse liquide contient au moins 40 % du poids total de la phase grasse liquide d'huile apolaire et mieux de 50 à 100 % du poids total de la phase grasse liquide.

18. Composition selon l'une des revendications précédentes, **caractérisée en ce que** la phase grasse liquide représente de 5 à 99 % du poids total de la composition et mieux de 20 à 75 %.

19. Composition selon l'une des revendications précédentes, **caractérisée en ce qu'**elle constitue une composition de soin et/ou de traitement et/ou de maquillage des matières kératiniques.

20. Composition selon l'une des revendications précédentes, **caractérisée en ce qu'**elle contient, en outre, au moins une matière colorante.

21. Composition selon la revendication 20, **caractérisée en ce que** la matière colorante est choisie parmi les colorants lipophiles, les colorants hydrophiles, les pigments, les nacres et leurs mélanges.

22. Composition selon la revendication 20 ou 21, **caractérisée en ce que** la matière colorante est présente à raison de 0,01 à 50 %, du poids total de la composition, de préférence de 5 à 30 %.

23. Composition selon l'une des revendications précédentes, **caractérisée en ce qu'**elle contient au moins un additif choisi parmi l'eau, les antioxydants, les huiles essentielles, les conservateurs, les parfums, les charges, les cires, les composés gras pâteux à température ambiante, les neutralisants, les polymères liposolubles ou dispersibles dans le milieu, les actifs cosmétiques ou dermatologiques, les dispersants, et leurs mélanges.

24. Composition selon l'une des revendications précédentes, **caractérisée en ce qu'**elle se présente sous forme d'un gel rigide, et notamment de stick anhydre.

25. Composition selon l'une des revendications précédentes, **caractérisée en ce qu'**elle se présente sous forme de mascara, d'eye-liner, de fond de teint, de rouge à lèvres, de blush, de produit déodorant ou démaquillant, de produit de maquillage du corps, de fard à paupières ou à joues, de produit anti- cerne, de shampoing, d'après shampoing, de protection solaire, de produit de soin du visage ou du corps.

26. Composition selon l'une des revendications précédentes, **caractérisée en ce qu'**elle constitue un produit de maquillage.

27. Procédé cosmétique de soin, de maquillage ou de traitement des matières kératiniques des êtres humains, comprenant l'application sur les matières kératiniques, d'une composition cosmétique conforme à l'une des revendications précédentes.

28. Utilisation de l'association d'au moins un solvant volatil choisi parmi les huiles de silicone linéaires ou cycliques ayant une viscosité à température ambiante inférieure à 8 cSt, les huiles volatiles hydrocarbonées ayant de 8 à 16 atomes de carbone et leurs mélanges, des solvants volatils fluorés, et leurs mélanges, d'au moins un polymère de masse moléculaire moyenne en poids allant de 1000 à 30 000, comportant a) un squelette polymérique ayant des motifs de répétition hydrocarbonés pourvus d'au moins un hétéroatome, et b) éventuellement au moins une chaîne grasse pendante et/ou terminale, éventuellement fonctionnalisée, le polymère étant un polyamide choisi parmi les polymères de formule (I) suivant et leurs mélanges : 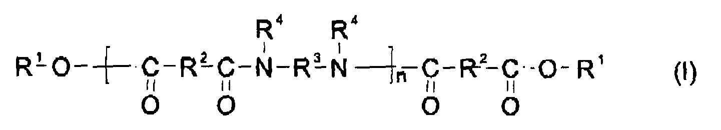 dans laquelle:
- n désigne un nombre de motifs amide tel que le nombre de groupes ester représente de 10% à 50% du nombre total des groupes ester et amide ;
- R¹ est à chaque occurrence indépendamment un groupe alkyle ou alcényle ayant au moins 4 atomes de carbones ;
- R² représente à chaque occurrence indépendamment un groupe hydrocarboné en C₄ à C₄₂ à condition que 50 % des groupes R² représentent un groupe hydrocarboné en C₃₀ à C₄₂;
- R³ représente à chaque occurrence indépendamment un groupe organique pourvus d'au moins 2 atomes de carbone, d'atomes hydrogène et optionnellement d'un ou plusieurs atomes d'oxygène ou d'azote ; et
- R⁴ représente à chaque occurrence indépendamment un atome d'hydrogène, un groupe alkyle en C₁ à C₁₀ ou une liaison directe à R³ ou un autre R⁴ de sorte quel'atome d'azote auxquels sont liés à la fois R³ et R⁴ fasse partie d'une structure hétérocyclique définie par R⁴-N-R³, avec au moins 50 % des R⁴ représentant un atome d'hydrogène, et
d'au moins un polymère liposoluble ou dispersible dans le milieu, présentant un poids moléculaire moyen de 500 à 1 000 000 et choisi parmi les polyalkylènes, les polyméthacrylates, les alkylcelluloses avec un radical alkyl linéaire ou ramifié, saturé ou non en C₁ à C₈, les polymères siliconés, les copolymères de vinylpyrrolidone, les polymères d'alcène en C₂ à C₃₀, et leurs associations,
dans une composition cosmétique ou pour la fabrication d'une composition physiologiquement acceptable, pour diminuer le transfert et/ou le dépôt de traces d'un film de ladite composition, appliqué sur les matières kératiniques, sur un support mis au contact dudit film, et/ou augmenter la tenue dudit film, et/ou diminuer sa migration.

29. Utilisation selon la revendication précédente, **caractérisée en ce que** le polymère est un polyamide comportant des groupements terminaux à groupe ester comportant une chaîne hydrocarbonée ayant de 10 à 42 atomes de carbone.

30. Utilisation selon la revendication 28 ou 29, **caractérisée en ce que** le solvant volatil est choisi parmi les isoparaffines en C₈-C₁₆, et notamment en C₈-C₁₃, d' isododécane et leurs mélanges.

## Claims

1. Structured cosmetic composition:
- containing at least one liquid fatty phase comprising at least one volatile solvent;
- said volatile solvent being chosen from linear or cyclic silicone oils having a viscosity at ambient temperature of less than 8 cSt, volatile hydrocarbon-based oils having from 8 to 16 carbon atoms, volatile fluorosolvents, and mixtures thereof with the exclusion of Isopar M;
- the liquid fatty phase being structured by at least one polymer having a weight-average molecular weight less than or equal to 100 000, comprising:
a) a polymer backbone, having hydrocarbon-based repeat units containing at least one heteroatom; and
b) at least one optionally functionalized pendent and/or terminal fatty chain having from 6 to 120 carbon atoms and being bonded to these hydrocarbon-based units,
the polymer being a polyamide chosen from the polymers of formula (I) below and mixtures thereof: 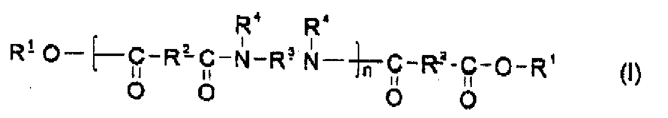 in which:
- n denotes a number of amide units such that the number of ester groups represents from 10 to 50% of the total number of ester and amide groups;
- R¹ is, independently in each case, an alkyl or alkenyl group having at least 4 carbon atoms;
- R² represents, independently in each case, a C₄ to C₄₂ hydrocarbon-based group on condition that 50% of the R² groups represent a C₃₀ to C₄₂ hydrocarbon-based group;
- R³ represents, independently in each case, an organic group containing at least 2 carbon atoms, hydrogen atoms and optionally one or more oxygen or nitrogen atoms; and
- R⁴ represents, independently in each case, a hydrogen atom, a C₁ to C₁₀ alkyl group or a direct bond to R³ or another R⁴ so that the nitrogen atom to which both R³ and R⁴ are bonded forms part of a heterocyclic structure defined by R⁴-N-R³, with at least 50% of the R⁴ groups representing a hydrogen atom,
the liquid fatty phase and the polymer forming a physiologically acceptable medium, the composition containing at least one polymer that is liposoluble or dispersible in the medium, having an average molecular weight of 500 to 1 000 000 and chosen from polyalkylenes, polymethacrylates, alkyl celluloses with a linear or branched, saturated or unsaturated C₁ to C₈ alkyl radical, silicone polymers, vinylpyrrolidone copolymers, C₂ to C₃₀ alkene copolymers, and combinations thereof.

2. Composition according to the preceding claim, **characterized in that** the fatty chains represent from 40 to 98% of the total number of units containing a heteroatom and of fatty chains.

3. Composition according to Claim 1 or 2, **characterized in that** the fatty chains represent from 50 to 95% of the total number of units containing a heteroatom and of fatty chains.

4. Composition according to one of the preceding claims, **characterized in that** the pendent fatty chains are directly bonded to at least one of said heteroatoms.

5. Composition according to one of the preceding claims, **characterized in that** the weight-average molecular weight ranges from 1000 to 30 000, preferably from 2000 to 20 000 and better still from 2000 to 10 000.

6. Composition according to one of the preceding claims, **characterized in that** the terminal fatty chains are bonded to the backbone by ester groups.

7. Composition according to one of the preceding claims, **characterized in that** the fatty chains have from 12 to 120 carbon atoms, and better still from 12 to 68 carbon atoms.

8. Composition according to one of the preceding claims, **characterized in that** R' is a C₁₂ to C₂₂ alkyl group.

9. Composition according to one of the preceding claims, **characterized in that** R² are groups having from 30 to 42 carbon atoms.

10. Composition according to one of the preceding claims, **characterized in that** the polymer represents from 0.5 to 80% of the total weight of the composition and better still from 5 to 40%.

11. Composition according to one of the preceding claims, in which the volatile solvent is chosen from oils that do not have a flashpoint, oils that have a flashpoint ranging from 40 to 100°C, and mixtures thereof.

12. Composition according to one of the preceding claims, **characterized in that** the volatile solvent is chosen from C₈-C₁₆ branched alkanes, C₈-C₁₆ branched esters, and mixtures thereof.

13. Composition according to one of the preceding claims, **characterized in that** the volatile solvent is chosen from C₈-C₁₆, especially C₈-C₁₃, isoparaffins, isododecane and mixtures thereof.

14. Composition according to one of the preceding claims, **characterized in that** the volatile solvent represents a mass content of 3 to 99.5%, preferably from 10 to 75% and better still from 15 to 45%.

15. Composition according to one of the preceding claims, **characterized in that** the liquid fatty phase contains, in addition, at least one non-volatile oil.

16. Composition according to one of the preceding claims, **characterized in that** the liquid fatty phase contains, in addition, at least one non-volatile oil chosen from hydrocarbon-based oils of mineral, plant or synthetic origin, synthetic esters or ethers, silicone oils and mixtures thereof.

17. Composition according to one of the preceding claims, **characterized in that** the liquid fatty phase contains at least 40% of the total weight of the liquid fatty phase, and better still from 50 to 100% of the total weight of the liquid fatty phase, of apolar oil.

18. Composition according to one of the preceding claims, **characterized in that** the liquid fatty phase represents from 5 to 99%, and better still from 20 to 75%, of the total weight of the composition.

19. Composition according to one of the preceding claims, **characterized in that** it constitutes a care and/or treatment and/or makeup composition for keratin materials.

20. Composition according to one of the preceding claims, **characterized in that** it contains, in addition, at least one dyestuff.

21. Composition according to Claim 20, **characterized in that** the dyestuff is chosen from lipophilic dyes, hydrophilic dyes, pigments, pearlescent agents and mixtures thereof.

22. Composition according to Claim 20 or 21, **characterized in that** the dyestuff is present in an amount of 0.01 to 50%, preferably 5 to 30%, of the total weight of the composition.

23. Composition according to one of the preceding claims, **characterized in that** it contains at least one additive chosen from water, antioxidants, essential oils, preservatives, fragrances, fillers, waxes, fatty compounds that are pasty at ambient temperature, neutralizers, polymers that are liposoluble or dispersible in the medium, cosmetic or dermatological active agents, dispersants, and mixtures thereof.

24. Composition according to one of the preceding claims, **characterized in that** it is in the form of a rigid gel, and in particular an anhydrous stick.

25. Composition according to one of the preceding claims, **characterized in that** it is in the form of mascara, eyeliner, foundation, lipstick, blusher, deodorant product or makeup-removing product, body makeup product, eyeshadow or face powder, concealer, shampoo, conditioner, sunscreen, or care products for the face or body.

26. Composition according to one of the preceding claims, **characterized in that** it constitutes a makeup product.

27. Cosmetic method of caring for, making up or treating the keratin materials of human beings, comprising the application to the keratin materials of a cosmetic composition conforming to one of the preceding claims.

28. Use of the combination of at least one volatile solvent chosen from linear or cyclic silicone oils having a viscosity at ambient temperature of less than 8 cSt, volatile hydrocarbon-based oils having from 8 to 16 carbon atoms and mixtures thereof, volatile fluorosolvents, and mixtures thereof, of at least one polymer having a weight-average molecular weight ranging from 1000 to 30 000, comprising a) a polymer backbone having hydrocarbon-based repeat units containing at least one heteroatom, and b) optionally at least one optionally functionalized pendent and/or terminal fatty chain, the polymer being a polyamide chosen from the polymers of formula (I) below and mixtures thereof: 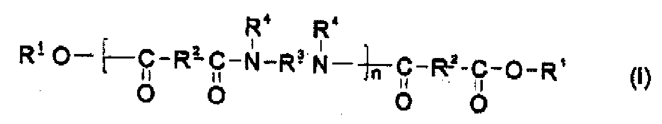 in which:
- n denotes a number of amide units such that the number of ester groups represents from 10 to 50% of the total number of ester and amide groups;
- R¹ is, independently in each case, an alkyl or alkenyl group having at least 4 carbon atoms;
- R² represents, independently in each case, a C₄ to C₄₂ hydrocarbon-based group on condition that 50% of the R² groups represent a C₃₀ to C₄₂ hydrocarbon-based group;
- R³ represents, independently in each case, an organic group containing at least 2 carbon atoms, hydrogen atoms and optionally one or more oxygen or nitrogen atoms; and
- R⁴ represents, independently in each case, a hydrogen atom, a C₁ to C₁₀ alkyl group or a direct bond to R³ or another R⁴ so that the nitrogen atom to which both R³ and R⁴ are bonded forms part of a heterocyclic structure defined by R⁴-N-R³, with at least 50% of the R⁴ groups representing a hydrogen atom, and
of at least one polymer that is liposoluble or dispersible in the medium, having an average molecular weight of 500 to 1 000 000 and chosen from polyalkylenes, polymethacrylates, alkyl celluloses with a linear or branched, saturated or unsaturated C₁ to C₈ alkyl radical, silicone polymers, vinylpyrrolidone copolymers, C₂ to C₃₀ alkene polymers, and combinations thereof,
in a cosmetic composition or for the manufacture of a physiologically acceptable composition, in order to reduce the transfer and/or deposit of traces of a film of said composition, applied to the keratin materials, onto a support placed in contact with said film, and/or to improve the staying power of said film, and/or to reduce its migration.

29. Use according to the preceding claim, **characterized in that** the polymer is a polyamide comprising terminal groups having an ester group comprising a hydrocarbon-based chain having from 10 to 42 carbon atoms.

30. Use according to Claim 28 or 29, **characterized in that** the volatile solvent is chosen from C₈-C₁₆, especially C₈-C₁₃, isoparaffins, isododecane and mixtures thereof.

## Patentansprüche

1. Strukturierte kosmetische Zusammensetzung
- enthaltend mindestens eine flüssige Fettphase, die mindestens ein flüchtiges Lösungsmittel einschließt,
- wobei das flüchtige Lösungsmittel ausgewählt ist aus linearen oder cyclischen Siliconölen mit einer Viskosität bei Umgebungstemperatur von unter 8 cSt, flüchtigen Kohlenwasserstoffölen mit 8 bis 16 Kohlenstoffatomen, flüchtigen fluorierten Lösungsmitteln und ihren Gemischen ausschließlich von Isopar M,
- wobei die flüssige Fettphase durch mindestens ein Polymer einer gewichtsmittleren Molmasse von kleiner oder gleich 100000 strukturiert ist,
- umfassend:
a) ein Polymerskelett mit Kohlenwasserstoff Wiederholungseinheiten, ausgestattet mit mindestens einem Heteroatom, und
b) mindestens eine angehängte und/oder terminale, gegebenenfalls funktionalisierte Fettsäurekette mit 6 bis 120 Kohlenstoffatomen und die an diese Kohlenwasserstoff-Einheiten gebunden ist,
wobei das Polymer ein Polyamid ist, das aus den Polymeren der folgenden Formel (I) und ihren Gemischen ausgewählt ist: 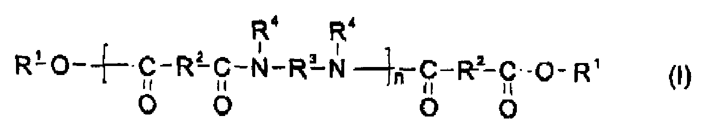
wobei:
- n eine Anzahl von Amid-Einheiten bezeichnet, derart, dass die Anzahl von Estergruppen 10 % bis 50 % der Gesamtanzahl der Ester- und Amidgruppen repräsentiert;
- R¹ in jedem Fall unabhängig eine Alkyl- oder Alkenylgruppe mit mindestens 4 Kohlenstoffatomen ist;
- R² in jedem Fall unabhängig eine C₄- bis C₄₂-Kohlenwasserstoffgruppe repräsentiert, mit der Maßgabe, dass 50 % der R²-Gruppen eine C₃₀- bis C₄₂-Kohlenwasserstoffgruppe repräsentieren;
- R³ in jedem Fall unabhängig eine organische Gruppe repräsentiert, ausgestattet mit mindestens zwei Kohlenstoffatomen, Wasserstoffatomen und gegebenenfalls einem oder mehreren Sauerstoff- oder Stickstoffatomen; und
- R⁴ in jedem Fall unabhängig ein Wasserstoffatom, eine C₁- bis C₁₀-Alkylgruppe oder eine direkte Bindung zu R³ oder einem anderen R⁴ repräsentiert, derart, dass das Stickstoffatom, an das gleichzeitig R³ und R⁴ gebunden sind, Teil einer heterocyclischen Struktur ausmacht, die durch R⁴-N-R³ definiert ist, wobei mindestens 50 % der R⁴ ein Wasserstoffatom darstellen,
wobei die Fettphase und das Polymer ein physiologisch verträgliches Medium bilden,
wobei die Zusammensetzung mindestens ein fettlösliches oder in dem Medium dispergierbares Polymer enthält, das ein mittleres Molekulargewicht von 500 bis 1000000 aufweist und ausgewählt ist aus Polyalkylenen, Polymethacrylaten, Alkylcellulosen mit einem linearen oder verzeigten, gesättigten oder ungesättigten C₁- bis C₈-Alkylrest, siliconierten Polymeren, Vinylpyrrolidon-Copolymeren, C₂- bis C₃₀-Alken-Copolymeren und ihren Kombinationen.

2. Zusammensetzung nach einem der vorhergehenden Ansprüche, **dadurch gekennzeichnet, dass** die Fettsäureketten 40 bis 98 % der Gesamtanzahl der Heteroatom-Einheiten und der Fettsäureketten repräsentieren.

3. Zusammensetzung nach Anspruch 1 oder 2, **dadurch gekennzeichnet, dass** die Fettsäureketten 50 bis 95 % der Gesamtanzahl der Heteroatom-Einheiten und der Fettsäureketten repräsentieren.

4. Zusammensetzung nach einem der vorhergehenden Ansprüche, **dadurch gekennzeichnet, dass** die angehängten Fettsäureketten direkt an mindestens eines der Heteroatome gebunden sind.

5. Zusammensetzung nach einem der vorhergehenden Ansprüche, **dadurch gekennzeichnet, dass** die gewichtsmittlere Molmasse von 1000 bis 30000, vorzugsweise von 2000 bis 20000 und besser von 2000 bis 10000 reicht.

6. Zusammensetzung nach einem der vorhergehenden Ansprüche, **dadurch gekennzeichnet, dass** die terminalen Fettsäureketten über Estergruppen an das Skelett gebunden sind.

7. Zusammensetzung nach einem der vorhergehenden Ansprüche, **dadurch gekennzeichnet, dass** die Fettsäureketten 12 bis 120 Kohlenstoffatome und besser 12 bis 68 Kohlenstoffatome aufweisen.

8. Zusammensetzung nach einem der vorhergehenden Ansprüche, **dadurch gekennzeichnet, dass** R' eine C₁₂- bis C₂₂-Alkylgruppe ist.

9. Zusammensetzung nach einem der vorhergehenden Ansprüche, **dadurch gekennzeichnet, dass** R2 Gruppen mit 30 bis 42 Kohlenstoffatomen sind.

10. Zusammensetzung nach einem der vorhergehenden Ansprüche, **dadurch gekennzeichnet, dass** das Polymer 0,5 bis 80 % des Gesamtgewichts der Zusammensetzung und besser 5 bis 40 % repräsentiert.

11. Zusammensetzung nach einem der vorhergehenden Ansprüche, wobei das flüchtige Lösungsmittel aus Ölen ohne Klarpunkt, Ölen mit einem Klarpunkt von 40 bis 100 °C und ihren Gemischen ausgewählt ist.

12. Zusammensetzung nach einem der vorhergehenden Ansprüche, **dadurch gekennzeichnet, dass** das flüchtige Lösungsmittel aus verzweigten C₈- bis C₁₆-Alkanen, verzweigten C₈- bis C₁₆-Estern und ihren Gemischen ausgewählt ist.

13. Zusammensetzung nach einem der vorhergehenden Ansprüche, **dadurch gekennzeichnet, dass** das flüchtige Lösungsmittel aus C₈- bis C₁₆- und insbesondere C₈- bis C₁₃- Isoparaffinen, Isododecan und ihren Gemischen ausgewählt ist.

14. Zusammensetzung nach einem der vorhergehenden Ansprüche, **dadurch gekennzeichnet, dass** das flüchtige Lösungsmittel einen Massegehalt von 3 bis 99,5 %, vorzugsweise von 10 bis 75 % und besser von 15 bis 45 % repräsentiert.

15. Zusammensetzung nach einem der vorhergehenden Ansprüche, **dadurch gekennzeichnet, dass** die flüssige Fettphase darüber hinaus mindestens ein nichtflüchtiges Öl enthält.

16. Zusammensetzung nach einem der vorhergehenden Ansprüche, **dadurch gekennzeichnet, dass** die flüssige Fettphase darüber hinaus ein nichtflüchtiges Öl enthält, das aus Kohlenwasserstoffölen mineralischen, pflanzlichen oder synthesischen Ursprungs, Synthese-Estem oder -Ethem, Siliconölen und ihren Gemischen ausgewählt ist.

17. Zusammensetzung nach einem der vorhergehenden Ansprüche, **dadurch gekennzeichnet, dass** die flüssige Fettphase mindestens 40 % des Gesamtgewicht der flüssigen Fettphase und besser von 50 bis 100 % des Gesamtgewichts der flüssigen Fettphase an apolarem Öl enthält.

18. Zusammensetzung nach einem der vorhergehenden Ansprüche, **dadurch gekennzeichnet, dass** die flüssige Fettphase 5 bis 99 % des Gesamtgewicht der Zusammensetzung und besser 20 bis 75 % enthält.

19. Zusammensetzung nach einem der vorhergehenden Ansprüche, **dadurch gekennzeichnet, dass** sie eine Pflege- und/oder Behandlungs- und/oder Schminkzusammensetzung der Keratinsubstanzen darstellt.

20. Zusammensetzung nach einem der vorhergehenden Ansprüche, **dadurch gekennzeichnet, dass** sie darüber hinaus mindestens einen Farbstoff enthält.

21. Zusammensetzung nach Anspruch 20, **dadurch gekennzeichnet, dass** der Farbstoff aus lipophilen Farbstoffen, hydrophilen Farbstoffen, Pigmenten, Perlmutt und ihren Gemischen ausgewählt ist.

22. Zusammensetzung nach Anspruch 20 oder 21, **dadurch gekennzeichnet, dass** der Farbstoff in einem Verhältnis von 0,01 bis 50 % des Gesamtgewichts der Zusammensetzung, vorzugsweise von 5 bis 30 % vorhanden ist.

23. Zusammensetzung nach einem der vorhergehenden Ansprüche, **dadurch gekennzeichnet, dass** sie mindestens ein Additiv enthält, ausgewählt aus Wasser, Antioxidantien, ätherischen Ölen, Konservierungsstoffen, Duftstoffen, Füllstoffen, Wachsen, bei Umgebungstemperatur pastösen Fettverbindungen, Neutralisationsmitteln, fettlöslichen oder in dem Medium dispergierbaren Polymeren, kosmetischen oder dermatologischen Wirkstoffen, Dispergiermitteln und ihren Gemischen.

24. Zusammensetzung nach einem der vorhergehenden Ansprüche, **dadurch gekennzeichnet, dass** sie in Form eines starren Gels und insbesondere eines wasserfreien Stifts vorliegt.

25. Zusammensetzung nach einem der vorhergehenden Ansprüche, **dadurch gekennzeichnet, dass** sie in Form von Mascara, Eyeliner, Fond de Teint, Lippenrot, Rouge, einem desodorierenden Produkt oder Abschminkprodukt, Körperschminkprodukt, Lidschatten oder Wagenrot, Antifaltenprodukt, Haarwäsche-, Nachschampoon-Produkt, Sonnenschutz, Gesicht- oder Körperpflegeprodukt vorliegt.

26. Zusammensetzung nach einem der vorhergehenden Ansprüche, **dadurch gekennzeichnet, dass** sie ein Schminkprodukt darstellt.

27. Kosmetisches Pflege-, Schmink- oder Behandlungsverfahren der Keratinsubstanzen der Menschen, umfassend das Aufbringen einer kosmetischen Zusammensetzung nach einem der vorhergehenden Ansprüche auf die Keratinsubstanzen.

28. Verwendung der Kombination von mindestens einem flüchtigen Lösungsmittel, ausgewählt aus linearen oder cyclischen Siliconölen mit einer Viskosität bei Umgebungstemperatur von unter 8 cSt, wobei die flüchtigen Kohlenwasserstofföle 8 bis 16 Kohlenstoffatome besitzen, und ihren Gemischen, fluorierten flüchtigen Lösungsmitteln und ihren Gemischen; von mindestens einem Polymer mit einer gewichtsmittleren Molmasse von 1000 bis 30000, umfassend: a) ein Polymerskelett mit Kohlenwasserstoff-Wiederholungseinheiten, ausgestattet mit mindestens einem Heteroatom und b) gegebenenfalls mindestens eine angehängte oder terminale, gegebenenfalls funktionalisierte Fettsäurekette, wobei das Polymer ein Polyamid ist, ausgewählt aus den Polymeren der folgenden Formel (I) und ihren Gemischen: 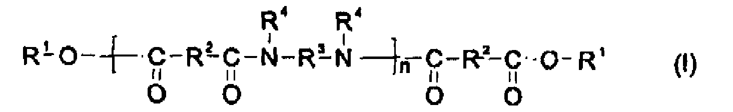 wobei:
- n eine Anzahl von Amid-Einheiten bezeichnet, derart, dass die Anzahl von Estergruppen 10 % bis 50 % der Gesamtanzahl der Ester- und Amidgruppen repräsentiert;
- R¹ in jedem Fall unabhängig eine Alkyl- oder Alkenylgruppe mit mindestens 4 Kohlenstoffatomen ist;
- R² in jedem Fall unabhängig eine C₄- bis C₄₂-Kohlenwasserstoffgruppe repräsentiert, mit der Maßgabe, dass 50 % der R²-Gruppen eine C₃₀- bis C₄₂-Kohlenwasserstoffgruppe repräsentieren;
- R³ in jedem Fall unabhängig eine organische Gruppe repräsentiert, ausgestattet mit mindestens zwei Kohlenstoffatomen, Wasserstoffatomen und gegebenenfalls einem oder mehreren Sauerstoff- oder Stickstoffatomen; und R⁴ in jedem Fall unabhängig ein Wasserstoffatom, eine C₁- bis C₁₀-Alkylgruppe oder eine direkte Bindung zu R³ oder einem anderen R⁴ repräsentiert, derart, dass das Stickstoffatom, an das gleichzeitig R³ und R⁴ gebunden sind, Teil einer heterocyclischen Struktur ausmacht, die durch R⁴-N-R³ definiert ist, wobei mindestens 50 % der R⁴ ein Wasserstoffatom darstellen, und
von mindestens einem fettlöslichen oder in dem Medium dispergierbaren Polymer, das ein mittleres Molekulargewicht von 500 bis 1000000 aufweist und ausgewählt ist aus Polyalkylenen, Polymethacrylaten, Alkylcellulosen mit einem linearen oder verzeigten, gesättigten oder ungesättigten C₁- bis C₈-Alkylrest, siliconierten Polymeren, Vinylpyrrolidon-Copolymeren, C₂- bis C₃₀-Alkenpolymeren und ihren Kombinationen,
in einer kosmetischen Zusammensetzung oder zur Herstellung einer physiologisch verträglichen Zusammensetzung zur Verminderung der Übertragung und/oder der Abscheidung von Spuren eines auf die Keratinsubstanzen aufgebrachten Films der Zusammensetzung auf einen Träger, der mit dem Film in Kontakt gebracht wird, und/oder zum Erhöhen des Gehalts des Films und/oder Vermindern seiner Migration.

29. Verwendung nach dem vorhergehenden Anspruch, **dadurch gekennzeichnet, dass** das Polymer ein Polyamid ist, das terminale Estergruppen-Gruppierungen umfasst, die eine Kohienwasserstofflcette mit 10 bis 42 Kohlenstoffatomen einschließen.

30. Verwendung nach Anspruch 28 oder 29, **dadurch gekennzeichnet, dass** das flüchtige Lösungsmittel aus C₈- bis C₁₆- und insbesondere C₈- bis C₁₃-Isoparaffinen, Isododecan und ihren Gemischen ausgewählt ist.
